# FASCICULE DE BREVET EUROPEEN

(11) **EP 3 482 021 B1**
(45) Date de publication et mention de la délivrance du brevet: **14.04.2021**
(21) Numéro de dépôt: 17748629.7
(22) Date de dépôt: 04.07.2017
(51) Int. Cl.: E04H 1/00, E04H 1/12, E04H 3/08

(54) **UNITÉ AUTONOME DE TRAITEMENT POUR DES SOINS PAR HÉMODIALYSE**
EIGENSTÄNDIGE BEHANDLUNGSEINHEIT FÜR HÄMODIALYSEBEHANDLUNGEN
SELF-CONTAINED TREATMENT UNIT FOR HAEMODIALYSIS

(30) Priorité: 05.07.2016 CH 8522016
(43) Date de publication de la demande: 15.05.2019
(73) Titulaire: Hemo Plus Sàrl, 1003 Lausanne (CH)
(72) Inventeur: GAUTHIER, Henri, 31000 Toulouse (FR); TILATTI, Nicolas, 47550 Boe (FR); COURTIADE, Philippe, 31280 Aigrefeuille (FR)
(74) Mandataire: Nithardt, Roland
(86) Numéro de dépôt international: PCT/CH2017/000070
(87) Numéro de publication internationale: WO 2018/006184

(56) Documents cités:
- WO-A1-03/095765
- WO-A1-2004/003934
- WO-A1-2010/004148
- DE-U1-202013 102 296

## Description

### Domaine technique

La présente invention concerne un centre de traitement pour traiter par hémodialyse, simultanément ou successivement, une pluralité de patients, ledit centre comportant au moins une unité de soins équipée d'un générateur de dialyse, ledit générateur de dialyse étant agencé pour préparer une solution de traitement, adaptée à chacun desdits patients de ladite pluralité de patients, ladite solution de traitement étant préparée par dilution avec de l'eau pour hémodialyse d'au moins un concentré composé de composés solides solubles dans l'eau, lesdits composés solides comprenant au moins du chlorure de sodium (NaCl), du chlorure de Potassium (KCl), du chlorure de calcium (CaCl₂), et du chlorure de Magnésium (MgCl₂).

### Technique antérieure

Les centres de soins de traitement de patients par hémodialyse sont souvent intégrés dans un hôpital ou une clinique, sous la forme d'un département affecté à ce traitement. Il s'avère que ces départements sont structurés de manière différente selon les hôpitaux et que l'organisation administrative et le suivi médical des patients doivent être conçus cas par cas. En outre, selon les centres médicaux concernés, l'espace disponible est variable, de sorte que les services annexes, tels que le stockage des produits de base, le traitement de l'eau destinée à préparer les solutions pour l'hémodialyse à partir de concentrés, le traitement et l'élimination des déchets sont dispersés en raison de leur diversification, ce qui complique à la fois le suivi des traitements et la sécurité des patients.

Or, le traitement de patients par hémodialyse est une opération répétitive de longue durée, étant donné qu'un patient dialysé est habituellement soigné au moins deux fois par semaine et que les interventions se déroulent pendant des mois voire des années, souvent à vie, quasiment de la même manière. Ceci implique que chaque soin nécessite sensiblement les mêmes opérations, les mêmes préparations, le même contrôle avant, pendant et après le traitement, de sorte qu'une rationalisation des démarches ainsi qu'une standardisation des équipements s'avère utile, à la fois pour simplifier les démarches, mieux les contrôler et les surveiller, mieux assurer la sécurité du patient et mieux constituer et/ou compléter le dossier médical de chacun des patients pour pouvoir disposer en permanence d'un dossier actualisé de chaque patient

Des unités autonomes de traitement médicaux sont connues par exemple des documents DE 20 2013 102296 U1, WO 03/095765 A1 et WO 2010/004148 A1.

Dans de grands centres médicaux, les affections soignées sont nombreuses et les services annexes doivent être largement diversifiés pour répondre à la demande des différents services correspondant aux différentes affectations. Dans de plus petits services, parfois éloignés d'un centre important, on constate l'intérêt de pouvoir disposer sur place de l'ensemble des infrastructures requises pour assurer les soins. C'est notamment le cas des traitements par l'hémodialyse qui sont effectués dans de grands centres approvisionnés par des unités centrales de production de concentrés, en opposition à de petits centres où, pour des questions de rentabilité et d'efficacité une production locale de concentrés est recommandée voire imposée.

La présente invention permet d'atteindre ces objectifs en proposant une unité autonome de traitement selon la revendication 1 ci-jointe. On réalise donc un centre de soins pour le traitement de patients par hémodialyse, qui constitue un modèle parfaitement adapté à ce traitement, et qui est reproductible à n'importe quel endroit, tout en gardant une souplesse en ce qui consiste la taille de la zone de traitement des patients proprement dite, selon la situation géographique du site d'implantation, des données démographiques et des moyens disponibles localement.

Le centre de soins selon l'invention fait idéalement partie d'un groupe de centres de soins, sensiblement identiques, qui sont implantés sur un territoire se situant plus ou moins autour d'une unité de production de concentrés pour le traitement de patients par hémodialyse. Ce concept permet de simplifier et de rationaliser considérablement l'approvisionnement des centres de traitement, rend le traitement moins coûteux et moins risqué, en garantissant un approvisionnement régulier à la demande des unités de soins et une distribution grâce à laquelle les risques liés au transport sont réduits. Il permet également de rapprocher les centres de soins des malades dans la mesure où ils ne nécessitent pas de structures hospitalières ou cliniques préexistantes. Leur autonomie autorise une implantation à l'extérieur ou à l'intérieur de bâtiments, préexistants, mais sans statut médical particulier.

Toutefois, lorsque les unités de soins sont plus petites ou lorsqu'elles sont très éloignées par rapport au centre de production de concentrés, un service intégré peut être mis en place dans le centre de soins pour produire sur place les concentrés nécessaires.

### Exposé de l'invention

C'est pourquoi la présente invention propose de réaliser un centre de traitement capable d'apporter une solution à l'ensemble des problèmes évoqués ci-dessus.

Ce but est atteint par l'unité de traitement autonome selon la revendication 1 ci-jointe. Le préambule de la revendication 1 décrit que ladite unité de traitement autonome est construite dans une structure d'accueil regroupant une pluralité de modules élémentaires réalisés sensiblement à l'identique, juxtaposés et agencés intérieurement, individuellement ou en combinaison, pour former au moins un local de soins pour un traitement par hémodialyse de ladite pluralité de patients, et des locaux dédiés, agencés pour assurer respectivement l'accueil des patients, le suivi administratif des patients, le suivi médical des patients, le stockage des concentrés, la préparation et le contrôle des fluides utilisés dans ledit local de soins et/ou le retraitement et/ou l'élimination des déchets et effluents résiduels provenant du traitement des patients.

Une unité de soins comprend un local de soins pour un traitement par hémodialyse, qui est monté dans au moins un module élémentaire et contient au moins un siège ou un lit de traitement d'un patient, associé à un ensemble de composants techniques de traitement comprenant un générateur pour hémodialyse, un dispositif de raccordement à un réseau d'alimentation électrique, un dispositif de raccordement à un réseau d'informatique, un dispositif de raccordement à une alimentation en eau pour hémodialyse, un dispositif de raccordement à une alimentation en concentrés pour hémodialyse, et un dispositif de collecte de déchets.

Ledit générateur pour hémodialyse est de agencé pour réaliser une solution adaptée à un patient spécifique en prélevant une quantité prédéterminée d'au moins un concentré pour hémodialyse et un volume prédéterminé d'eau pour hémodialyse et pour effectuer le mélange de ces composants en vue de préparer et d'injecter ladite solution adaptée audit patient spécifique pendant la durée du traitement.

Ledit local de soins pour un traitement par hémodialyse est biplace et contient deux sièges ou lits de traitement disposés en regard pour le traitement simultané de deux patients, chacun desdits sièges ou lits étant associé à un ensemble de composants techniques de traitement.

De manière à assurer à la fois le transport et le montage de l'unité de soins, chaque module élémentaire comprend une ossature rigide sur laquelle sont montées des parois de fermeture et/ou de séparation, dans lequel ladite ossature rigide comporte, dans sa partie supérieure un espace vide pour contenir des composants techniques.

Ledit espace vide contient de préférence au moins un canal de distribution d'air purifié entrant et au moins un canal d'évacuation d'air ambiant sortant, ainsi qu'un circuit de circulation de liquide chauffé ou réfrigéré pour assurer une climatisation de l'intérieur dudit local de traitement.

Selon un mode de réalisation préféré, ledit local de soins est disposé sensiblement au centre de ladite structure d'accueil qui comprend d'une part un premier ensemble de modules élémentaires constituant des premiers locaux dédiés, assemblés pour être aménagés en local de réception des patients, en local de prise en charge administrative, en local de prise en charge médicale, et un deuxième ensemble de modules élémentaires constituant des seconds locaux dédiés, assemblés pour être aménagés en local de stockage des solutions de concentré, en local de traitement de l'eau, en local de stockage de produits pharmaceutiques, en local de gestion de l'énergie électrique et en local de traitement/élimination de déchets.

Ledit local de soins pour un traitement par hémodialyse comporte au moins un module élémentaire affecté à des traitements de patients ayant des contaminations particulières nécessitant des précautions médicales pour protéger les autres patients et/ou le personnel soignant, ledit module élémentaire ayant un accès sécurisé.

Ladite ossature rigide sur laquelle sont montées des parois de fermeture et/ou de séparation, est une ossature métallique et lesdites parois de fermeture et/ou de séparation sont réalisées avec des panneaux de matériaux composites thermiquement isolés couverts de matériaux de finition étanches.

Les ossatures rigides des modules élémentaires juxtaposés sont de préférence assemblées de telle manière que chaque local dédié présente une surface adaptée à sa fonction et les parois de fermeture /séparation sont montées sur lesdites ossatures rigides de manière à délimiter géométriquement ladite surface adaptée à la fonction dédiée audit local.

Chacun des modules élémentaires comporte un double plafond qui définit ledit espace libre en haut des locaux de soins et des locaux dédiés, ledit espace libre étant affecté à des conduits et/ ou des gaines techniques.

Ledit local technique comporte avantageusement au moins des moyens de réception et de stockage des matières premières, une unité de prélèvement, des moyens de pesée, un équipement de préparation des solutions de traitement des patients, une unité de filtration et une unité de remplissage de conteneurs destinés à stocker les concentrés.

### Description sommaire des dessins

La présente invention et ses avantages apparaîtront mieux dans la description ci-dessous d'un mode de réalisation donné à titre d'exemple non limitatif, en référence aux dessins annexés dans lesquels :
- la figure 1 est une vue d'ensemble schématique représentant une forme de réalisation d'une unité de soins pour hémodialyse selon l'invention,
- les figures 2A et 2B représentent des vues en coupe partielle d'un module élémentaire illustrant le mode de construction de l'ossature rigide et des parois constituant les cloisons extérieures de ce module,
- la figure 3 est une vue en perspective d'une forme de réalisation préférée d'un local basique de soins pour une unité de soins selon l'invention, et
- la figure 4 est un diagramme de fonctionnement d'une unité de soins pour hémodialyse combinée avec un service dédié de fabrication des produits de traitement.

### Meilleure manière de réaliser l'invention

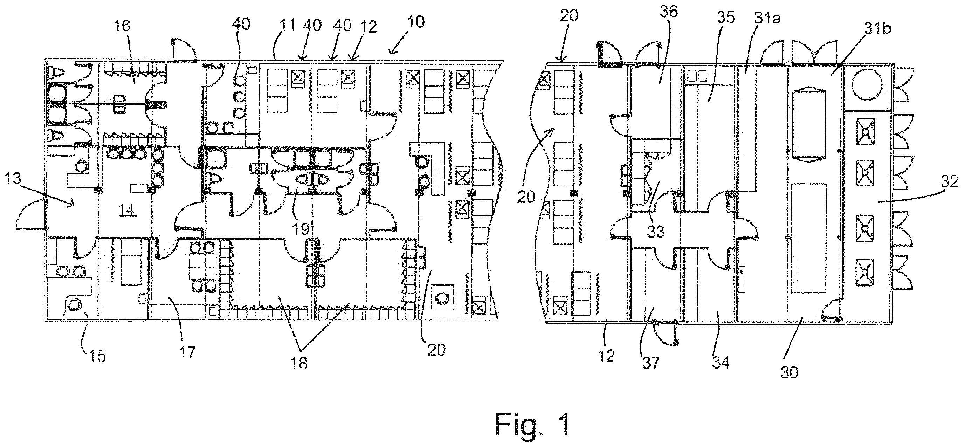

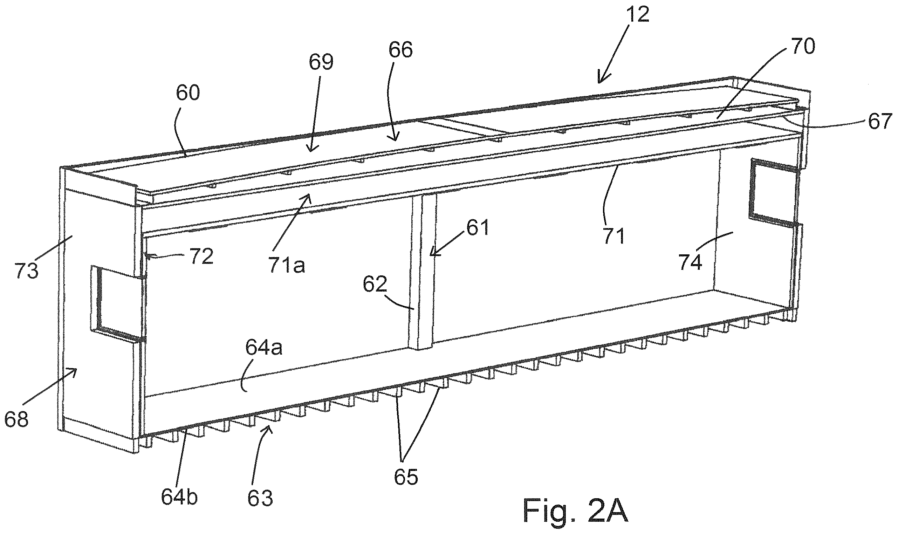

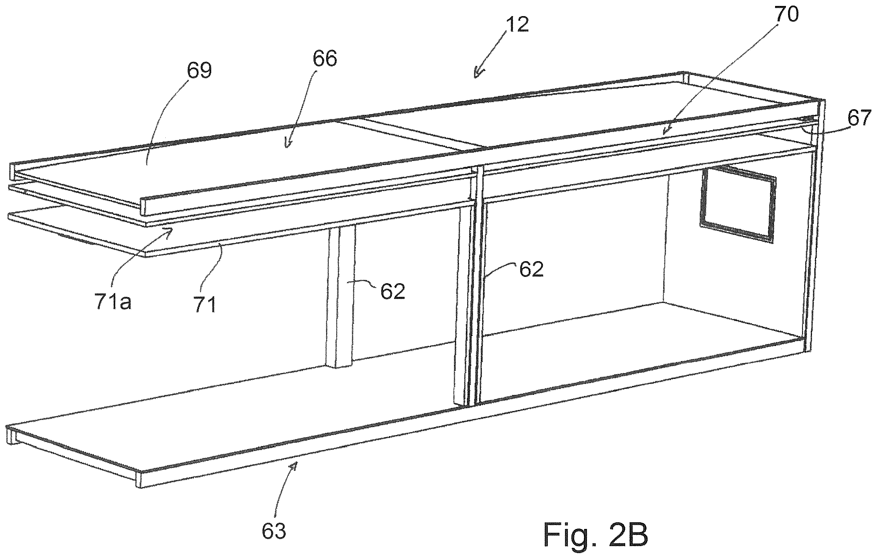

En référence aux figures 1 à 3, notamment de la figure 1, le centre 10 de traitement de patients par hémodialyse, décrit ci-dessous, est construit dans une structure d'accueil 11 regroupant une pluralité de modules élémentaires 12 réalisés sensiblement à l'identique. Les modules élémentaires 12 sont représentés plus en détails par les figures 2A et 2B. Ils sont juxtaposés dans le sens de la largeur ou dans le sens de la longueur et peuvent être agencés intérieurement, individuellement ou en combinaison, pour former d'une part, une ou plusieurs salles de soins pour un traitement de patients par hémodialyse, et d'autre part des locaux dédiés, agencés pour assurer respectivement l'accueil des patients, le suivi administratif des patients, le suivi médical des patients, le stockage des matières premières, la préparation individuelle des solutions de traitement adaptées aux patients, la préparation et le contrôle des fluides utilisés dans ledit local de soins et/ou le retraitement et/ou l'élimination des déchets et/ou des effluents, issus notamment dudit traitement des patients.

La salle de soins 20 est avantageusement composée de cellules individuelles ou doubles qui sont ménagées dans un ou plusieurs modules élémentaires 12. Ces modules élémentaires 12 peuvent être adaptés aux besoins en fonction de l'implantation des centres de traitement, de la géographie des sites d'implantation et/ou des données relatives à la population et des conditions locales.

La figure 1 représente un centre 10 de traitement de patients par hémodialyse qui comprend un bâtiment ayant une forme sensiblement rectangulaire, appelé structure d'accueil 11, et qui est montée de préférence sur une dalle plane servant de support à une pluralité de modules élémentaires 12. Les modules élémentaires 12 sont avantageusement des caissons ou des conteneurs (voir figures 2A et 2B) ou similaires, de forme parallélépipédique, dont les dimensions sont adaptées pour permettre un transport simple et un aménagement économique, tout en restant efficace, et pour permettre l'installation, sous une forme individuelle ou groupée de tous les équipements requis. Ces équipements sont d'une part prévus et conçus pour constituer au moins une salle de soins 20 destinée au traitement de patients ou au moins un local dédié 30, destiné à regrouper les composants appropriés à une ou plusieurs fonctions annexes, indispensables avant, pendant ou après le traitement des patients.

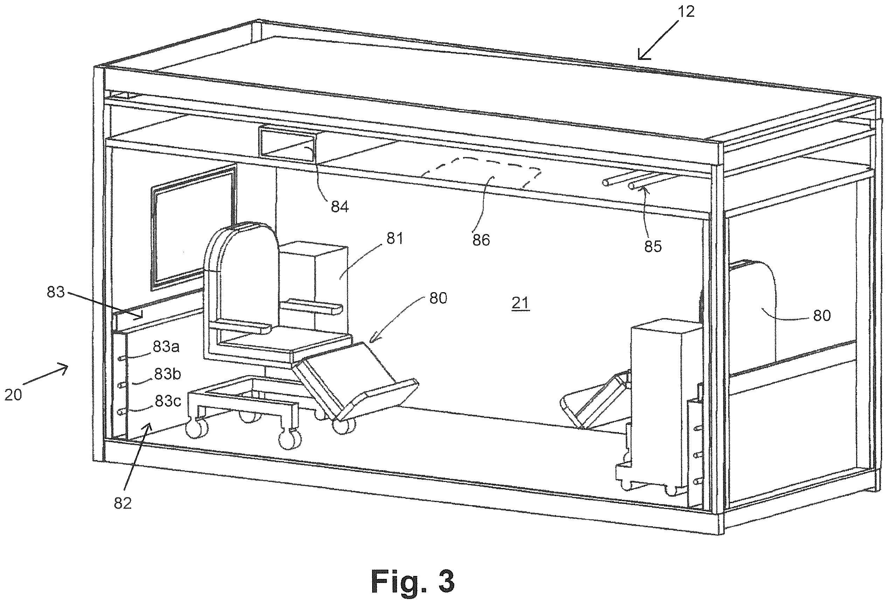

L'unité de traitement 10, dans son ensemble, est aménagée autour d'une partie centrale, constituée de la salle de soins 20, composée d'une ou de plusieurs cellules de soins basiques 21. Une cellule de soins basique 21 est illustrée et sera décrite en détail en référence à la figure 3. Dans l'exemple représenté, la salle de soins 20 comporte au moins une subdivision 40 qui est affectée à des patients nécessitant des précautions particulières, notamment des patients contaminés par des virus dangereux, qui risquent de se propager et de contaminer le personnel soignant et/ou d'autres patients.

L'accès à la salle de soins est réservé aux patients qui ont été préalablement reçus dans une zone d'accueil 13 par un personnel de réception. La zone d'accueil est organisée pour assurer la gestion administrative des patients, à savoir les démarches d'enregistrement, les prises de rendez-vous et pour les diriger vers une salle d'attente 14. Par la suite les patients sont pris en charge par une division médicale 15 qui est agencée pour effectuer les analyses médicales requises et pour assurer le suivi médical, pour que toutes les éventuelles démarches médicales préparatoires requises soient effectuées avant le début d'une séquence de traitement en salle de soins.

A proximité de la zone d'accueil 13, en amont de la salle de soins 12, sont également installées différentes zones affectées au personnel soignant, comme par exemple un vestiaire et un local de préparation 16, une cantine ou restaurant d'entreprise 17 et des locaux annexes 18 pour permettre aux patients de se changer et de revêtir les blouses de soins ou similaires, une salle d'équipements sanitaires 19 et une salle de repos 40, indispensable pour que les patients puissent y séjourner, pour une période donnée, après leur traitement.

Les patients subissent le traitement dans une des cellules de soins basique 21 ou dans l'une des subdivisions spéciales 40 de la salle de soins 20, en fonction des décisions prises par la division médicale. La préparation des solutions de traitement, la gestion des fluides et des températures, l'approvisionnement en énergie électrique, l'approvisionnement et le traitement de l'eau pour dialyse, la gestion des stocks de fluides et de produits pharmaceutiques sont organisés dans une autre partie du centre 10, de préférence dans des locaux dédiés 30 qui sont installés dans des modules élémentaires 12, couplés et combinés entre eux, installés en locaux spécifiquement dédiés aux différentes fonctions définies ci-dessus. Le couplage et la combinaison des modules élémentaires 12 sont effectués par la mise en place de parois de séparation qui permettent de séparer les espaces requis pour remplir les fonctions. L'ensemble desdits locaux dédiés que l'on pourrait également qualifier de locaux de service est avantageusement disposé de l'autre côté de la salle de soins 20, par rapport aux services d'accueil et de préparation médicale des patients.

Parmi les locaux regroupés sous la dénomination générale de locaux dédiés 30 on peut mentionner :
- un local 31a comportant des moyens de traitement de l'eau ;
- un local 31b pour le stockage d'eau traitée pour garantir une autonomie suffisante de l'unité de soins 10 ;
- un local 32 qui regroupe les stocks de concentrés, destinés à être dilués par des générateurs pour hémodialyse, et servant de base pour tous les traitements par hémodialyse ;
- un local 33 de stockage de produits pharmaceutiques susceptibles d'être utilisés avant, pendant ou après un traitement par un patient ;
- un local 34 de gestion et, en cas de défaillance d'un réseau d'alimentation en énergie électrique, de production d'énergie électrique ;
- un local 35 de stockage de produits chimiques, notamment pour le nettoyage et la désinfection des installations et des équipements ;
- un local 36 de traitement et/ou d'inertage et/ou d'élimination de déchets résultant du traitement ; et
- un local de stockage de pièces techniques pour assurer la maintenance des équipements.

Un équipement de production d'énergie électrique pourrait, pour des raisons de sécurité, être monté dans un local dédié 30 complémentaire, ayant la particularité d'être situé à l'extérieur. Il en est de même d'un équipement de production d'air purifié, d'un dispositif d'échanges thermiques destiné à générer, le cas échéant des fluides chauffés ou des fluides réfrigérés notamment pour le chauffage et/ou la climatisation. Ces équipements, pourraient, selon le cas, être montés dans des modules élémentaires complémentaires détachés de la structure d'accueil 11 du centre de traitement 10.

Les figures 2A et 2B représentent un module élémentaire 12, en perspective et en coupe longitudinale pour la figure 2A et ouvert à une extrémité pour la figure 2B. Un module élémentaire 12 est sensiblement conçu comme un conteneur 60 de forme parallélépipédique comportant une ossature rigide 61, faite par exemple avec des poutrelles métalliques 62 réalisées sous forme de profilés en acier galvanisé ou similaire, sur laquelle est monté d'une part un fond 63 sous forme d'un plancher en bois ou une plaque en béton vibré ou similaire 64a associé à une plaque de tôle anti-rongeurs 64b disposée vers l'extérieur, supporté par des renforts sous forme de poutrelles métalliques 65 et d'autre part un plafond 66, de préférence constitué d'une plaque de tôle galvanisée plane 67. Ensuite, selon les besoins de l'unité 10, un module élémentaire peut être utilisé de façon indépendante ou pris en combinaison avec d'autres modules élémentaires identiques et des parois de fermeture 68 ou des parois de séparation, qui peuvent être mis en place selon les besoins imposés par les aménagements prévus. Le toit du module élémentaire 12 est recouvert d'une tôle galvanisée 69, incurvée pour assurer l'écoulement de l'eau de pluie et disposée de manière à ménager un espace 70 par rapport à la plaque de tôle galvanisée 67 du plafond 66. Cet espace 70 est comblé par un matériau isolant, par exemple de la laine de verre de l'ordre de 200mm d'épaisseur, ou tout autre matériau isolant approprié.

On notera que le module élémentaire 12 est équipé d'un faux plafond 71, sous la forme d'un panneau parallèle qui définit avec la plaque de tôle galvanisée 67 du plafond 66, un vide technique 71a utilisable pour diverses installations, comme le réseau de circulation forcée d'air, les circuits de câblages électriques, la climatisation etc. Les parois latérales sont avantageusement constituées de panneaux multicouches 72 avec une couche extérieure 73 protectrice et résistant aux intempéries, une ou plusieurs couches intermédiaires en matériaux isolants comme par exemple la mousse de polyuréthane et un revêtement intérieur agréé pour des salles blanches. D'une manière générale tous les revêtements intérieurs, et plus spécifiquement tous les revêtements intérieurs 74 qui correspondent à la salle de soins ou aux locaux sensibles, sont faits avec des matériaux agréés pour effectuer le revêtement de salles blanches.

La figure 3 illustre une forme de réalisation d'une salle de soins 20 constituée d'au moins une cellule de soins basique 21 disposée dans un module élémentaire 12. Il s'agit en fait d'une salle de soins double, c'est-à-dire aménagée pour recevoir et traiter simultanément deux patients. A cet effet, elle comporte deux sièges de soins 80, qui pourraient être des lits ou des fauteuils, disposés aux deux extrémités longitudinales du module 12, et placés face à face. Ils peuvent être séparés par un rideau amovible ou par un paravent (non représentés), afin de respecter l'intimité de chacun des patients. A chacun desdits sièges 80 est associé un générateur d'hémodialyse 81. Derrière chaque siège est montée une goulotte technique 82 de distribution de fluides et une goulotte 83 de câbles qui permettent d'alimenter en énergie, en données de communication et en liquides utilisés pendant le traitement, d'une part le générateur de dialyse et d'autre part tous les appareils requis pendant ce traitement. La goulotte 82 contient avantageusement d'une part un circuit de soufflage d'air climatisé 84a et un circuit de reprise de cet air climatisé 84b. La goulotte 82 comporte par ailleurs une arrivée 83a d'eau pour hémodialyse, une arrivée 83b de concentrés de dialyse, une sortie 83c de liquide d'hémodialyse en vue de son évacuation. La goulotte de câblage 83 contient des câbles d'alimentation électrique du générateur d'hémodialyse 81 et des équipements électriques du siège 80. Des câbles de communication pour raccorder le siège 80 et le générateur d'hémodialyse à une centrale de gestion des données informatiques sont également logés dans ladite goulotte de câblage 83. Le générateur d'hémodialyse est conçu pour recevoir l'eau pour l'hémodialyse à l'arrivée 82a, le concentré à l'arrivée 82b ou alimenté par bidons de 5 ou 10 litres, qui peut par exemple, être différent de celui du réseau, pour être adapté au patient. La goulotte 82 a en outre la possibilité de communiquer, par des conduits appropriés (non représentés), avec une gaine de soufflage 84 et une bouche de reprise 86 ménagées dans le faux-plafond. A cet effet, la goulotte 82 contient un conduit d'amenée d'air 84a et un conduit de reprise 84b pour la régénération de l'air. Dans le faux-plafond sont disposés des circuits de d'eau réfrigérée ou réchauffée 85 qui permettent de rafraichir ou de réchauffer l'ai selon les besoins, et de l'évacuer par des bouches de sorties d'air climatisé 88, disposées dans le faux-plafond.

Par ailleurs, on notera que le faux-plafond 71 permet de créer ledit espace vide 70 qui contient un canal 84a de soufflage et de reprise d'air 84b dans le local de soins 20. Un circuit 85 de circulation d'eau réfrigérée ou réchauffée, permet de rafraichir ou réchauffer l'atmosphère ambiante dans le local de soins 20. Une bouche de sortie d'air climatisé 87 est ménagé dans la plaque de fermeture du faux-plafond 71. Un éclairage 86 est ménagé dans la plaque de fermeture du faux-plafond 71.

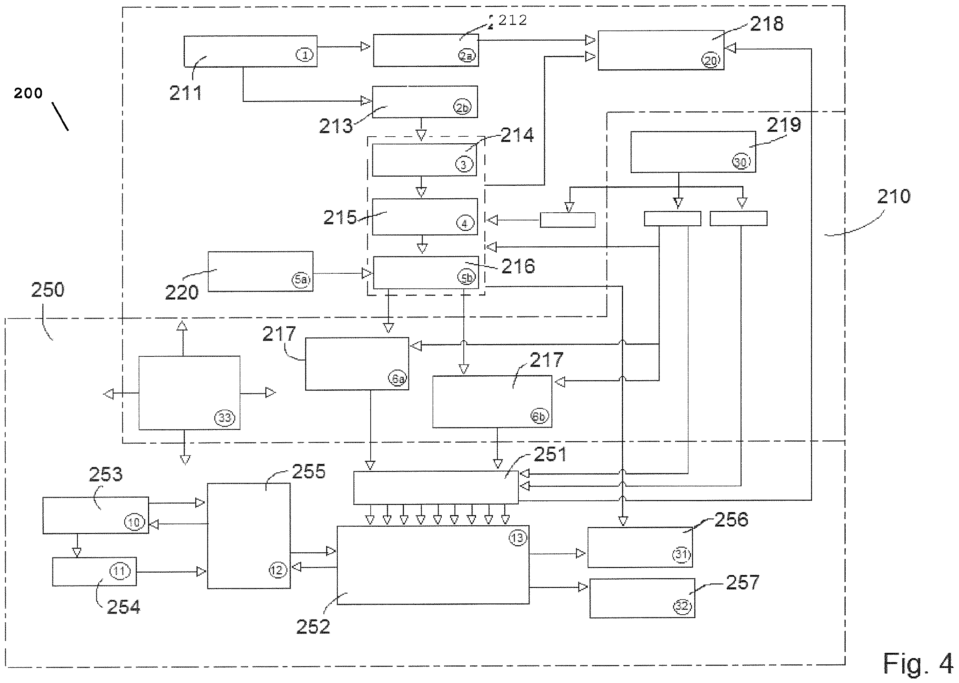

La figure 4 illustre une évolution du concept en vue d'une adaptation à des unités de traitement de patients par hémodialyse, plus petits ou isolés, qui comportent à la fois les structures permettant la préparation des produits de traitement et les structures adaptées à la prise en charge et au suivi des patients. A cet effet, le dispositif 200 illustré schématiquement se compose de deux entités 210 et 250 qui sont respectivement affectées à la préparation des produits et aux soins des patients. L'entité 210 de préparation des produits comprend des moyens 211 de réception et de stockage des matières premières, une unité de prélèvement 212, une pesée 213, un équipement de préparation 214, une unité de filtration 215 et une unité de remplissage 216 de conteneurs destinés à stocker les concentrés. Les concentrés peuvent être stockés en bidons ayant par exemple une contenance de 5 et de 10 litres, ou en cuves de 800 ou 1000 litres.

Attachés à ces services, on peut prévoir des moyens de stockage 217 des articles de conditionnement en vue de leur réutilisation après usage. Un laboratoire d'analyse 218 complète cet équipement pour garantir la qualité des substances préparées. Une unité de préparation d'eau purifiée 219 est intégrée à l'unité 210. Des moyens pour assurer le traitement de l'air 220 peuvent également être prévus. Ils peuvent être affectés aussi bien à l'unité de préparation des produits 210 qu'à l'unité de soins 250.

L'unité de soins 250 comprend principalement un générateur de dialyse 251 qui est directement couplé à une salle de soins 252, qui est par exemple une salle commune équipée de paravents ou de semi-cloisons internes. La salle de soins 252 est pourvue de fauteuils de traitement individuels, séparés par des rideaux ou par les paravents. Quelques lits individuels peuvent être prévus pour des cas spéciaux ou graves. A l'entrée de la salle de soins 252 se situe une structure d'accueil 253 pour assurer la réception des patients et leur orientation dans le centre. Un local 254 de consultation de médecins est adjointe à la réception 253. Un vestiaire 255 pour les patients est associé à l'accueil 253. A la sortie de la salle de soins 252 se situent des locaux techniques de neutralisation des effluents 256 et d'incinération des déchets 257. Le générateur de dialyse 251 est directement connecté aux moyens de stockage 217 des concentrés pour la dialyse ainsi qu'à l'unité de préparation d'eau purifiée 119 en vue d'adapter les produits de traitement en fonction des patients et en tenant compte des résultats des analyses.

L'invention n'est pas limitée aux formes de réalisation décrites et peut se présenter sous différents aspects en fonction de certaines évolutions prévisibles ou en fonction de besoins spécifiques. Les dimensions de la salle de soins, sa structuration, notamment la disposition des fauteuils de soins ou la disposition des locaux dédiés affectés aux différents services annexes, peuvent être modifiées en fonction des besoins ou des disponibilités locales. Néanmoins la portée de ces modifications se limite à des transformations évidentes pour un homme de l'art, dans le cadre de l'invention définit par les revendications ci-jointes.

En particulier la goulotte technique 82 peut être réalisée soit en tronçons partiels de la dimension de chaque module ou avoir la dimension de plusieurs modules avec des liaisons connectables entre modules, pour recevoir les différents circuits câblés électriques pour les courants forts et les courants faibles, les réseaux de fluides et les gaines pour les circuits aérauliques climatisés.

## Revendications

1. Unité autonome de traitement (10; 200) pour traiter par hémodialyse, au moins un patient, ladite unité (10; 200) comportant au moins une section de soins équipée d'un générateur de dialyse, ledit générateur de dialyse étant agencé pour préparer une solution de traitement, adaptée audit au moins un patient, ladite solution de traitement étant préparée par dilution avec de l'eau pour hémodialyse d'au moins un concentré composé de composés solides solubles dans l'eau, lesdits composés solides comprenant au moins du chlorure de sodium (NaCl), du chlorure de Potassium (KCl), du chlorure de calcium (CaCl₂), et du chlorure de Magnésium (MgCl₂),
ladite unité autonome de traitement (10; 200) est construite dans une structure d'accueil (11) regroupant une pluralité de modules élémentaires (12) réalisés sensiblement à l'identique, juxtaposés et agencés intérieurement, individuellement ou en combinaison, pour former un premier local de soins (20; 250) affecté aux soins pour un traitement par hémodialyse dudit au moins un patient, des locaux dédiés, agencés pour assurer respectivement l'accueil des patients, le suivi administratif des patients, le suivi médical des patients, et un second local technique (30; 210) pour la préparation et le stockage des concentrés, la préparation et le contrôle des fluides utilisés dans ledit premier local de soins (20; 250) et/ou le retraitement et/ou l'élimination des déchets et effluents résiduels provenant du traitement des patients,
ledit au moins un premier local de soins (20; 250) pour un traitement par hémodialyse est constitué d'au moins une cellule de soins basique (21; 252) et est monté dans au moins un module élémentaire (12) et contient au moins un siège (80) ou lit de traitement d'un patient, associé à un ensemble de composants techniques de traitement, lesdits composants techniques comprenant un générateur pour hémodialyse (81) agencé pour préparer une solution de traitement en diluant ledit au moins un concentré pour l'adapter audit patient à traiter, un dispositif de raccordement à un réseau d'alimentation électrique, un dispositif de raccordement à un réseau d'informatique, un dispositif de raccordement à une alimentation en eau pour hémodialyse, un dispositif de raccordement à une alimentation en concentré pour hémodialyse, et un dispositif de collecte de déchets,
et dans ladite au moins une cellule de soins basique (21; 252) ledit générateur pour hémodialyse (81) est agencé pour réaliser une solution adaptée à un patient spécifique en prélevant une quantité prédéterminée de concentrés pour hémodialyse et un volume prédéterminé d'eau pour hémodialyse et pour effectuer le mélange de ces composants en vue de préparer et d'injecter ladite solution adaptée audit patient spécifique pendant la durée du traitement,
**caractérisée en ce que**, ladite au moins une cellule de soins basique (21; 252) pour un traitement par hémodialyse est biplace et contient deux sièges (80) ou lits de traitement pour le traitement simultané de deux patients, chacun desdits sièges ou lits étant associé à un ensemble de composants techniques de traitement, et **en ce que**, chaque module élémentaire (12) comporte une ossature rigide (61) sur laquelle sont montées des parois de fermeture (68) et/ou de séparation, dans lequel ladite ossature rigide comporte, dans sa partie supérieure un espace vide (70) pour contenir des composants techniques.

2. Unité autonome de traitement (10) selon la revendication 1, **caractérisée en ce que** ledit espace vide (70) contient au moins un canal de distribution d'air purifié entrant et au moins un canal d'évacuation d'air ambiant sortant, ainsi qu'un circuit de circulation de liquide chauffé ou réfrigéré pour assurer une climatisation à l'intérieur dudit local de traitement.

3. Unité autonome de traitement (10) selon la revendication 1, **caractérisée en ce que** ledit local de soins (20) est disposé sensiblement au centre de ladite structure d'accueil (11) qui comprend d'une part un premier ensemble de modules élémentaires (12) constituant des premiers locaux dédiés, assemblés pour être aménagés en local de réception des patients, en local de prise en charge administrative, en local de prise en charge médicale, et un deuxième ensemble de modules élémentaires constituant des seconds locaux dédiés, assemblés pour être aménagés en local de stockage des solutions de concentré, en local de traitement de l'eau, en local de stockage de produits pharmaceutiques, en local de gestion de l'énergie électrique et en local de traitement/élimination de déchets.

4. Unité autonome de traitement (10) selon la revendication 1, **caractérisée en ce que** ledit local de soins (20) pour un traitement par hémodialyse comporte au moins un module élémentaire (12) affecté à des traitements de patients de patients ayant des contaminations particulières nécessitant des précautions médicales pour protéger les autres patients et/ou le personnel soignant, ledit module élémentaire ayant un accès sécurisé.

5. Unité autonome de traitement (10) selon la revendication 1, **caractérisée en ce que** ladite ossature rigide (61) sur laquelle sont montées des parois de fermeture et/ou de séparation, est une ossature métallique et **en ce que** lesdites parois de fermeture (68) et/ou de séparation sont réalisées avec des panneaux de matériaux composites thermiquement isolés couverts de matériaux de finition étanches.

6. Unité autonome de traitement (10) selon la revendication 1, **caractérisée en ce que** les ossatures rigides (61) des modules élémentaires (12) juxtaposés sont assemblées de telle manière que chaque local dédié présente une surface adaptée à sa fonction et les parois de fermeture et/ou de séparation sont montées sur lesdites ossatures rigides de manière à délimiter géométriquement ladite surface adaptée à la fonction dédiée audit local.

7. Unité autonome de traitement (10) selon la revendication 1, **caractérisée en ce que** chacun des modules élémentaires (12) comporte un double plafond (71) qui définit ledit espace libre (70) en haut des locaux de soins et des locaux dédiés, ledit espace libre (70) étant affecté à des conduits et/ou des gaines techniques (84, 85).

8. Unité autonome de traitement (200) selon la revendication 1, **caractérisée en ce que** ledit local technique (210) comporte des moyens (211) de réception et de stockage des matières premières, une unité de prélèvement (212), des moyens de pesée (213), un équipement de préparation des solutions de traitement des patients (214), une unité de filtration (215) et une unité de remplissage (216) de conteneurs destinés à stocker les concentrés,

9. Unité autonome de traitement (200) selon la revendication 1, **caractérisée en ce que** ledit local technique (210) est intégré dans le local de traitement (250).

## Patentansprüche

1. Autonome Behandlungseinheit (10; 200) zum Behandeln von mindestens einem Patienten durch Hämodialyse, wobei die Einheit (10; 200) mindestens einen mit einem Dialysegenerator ausgestatteten Pflegeabschnitt umfasst, wobei der Dialysegenerator zum Herstellen einer für mindestens einen Patienten geeigneten Behandlungslösung eingerichtet ist, wobei die Behandlungslösung durch Verdünnen mit Wasser zur Hämodialyse von mindestens einem Konzentrat hergestellt wird, das aus in Wasser löslichen festen Verbindungen besteht, wobei die festen Verbindungen mindestens Natriumchlorid (NaCI), Kaliumchlorid (KCl) Calciumchlorid (CaCl2) und Magnesiumchlorid (MgCl2) umfassen, wobei die autonome Behandlungseinheit (10; 200) in einer Empfangsstruktur (11) aufgebaut ist, die mehrere Grundmodule (12) zusammenfasst, die im Wesentlichen identisch ausgeführt, nebeneinander liegend und einzeln oder in Kombination im Inneren eingerichtet sind, um einen ersten Pflegeraum (20; 250), der zur Pflege für eine Behandlung durch Hämodialyse von mindestens einem Patienten vorgesehen ist, spezielle Räume, die so eingerichtet sind, um jeweils die Aufnahme von Patienten, die administrative Betreuung der Patienten, die medizinische Betreuung der Patienten zu gewährleisten, und einen zweiten Technikraum (30; 210) zum Herstellen und Lagern von Konzentraten, zum Herstellen und Kontrollieren der in dem ersten Pflegeraum (20; 250) verwendeten Fluide und/oder zum Wiederaufbereiten und/oder Entsorgen von Abfällen und Restabwässern aus der Behandlung von Patienten zu bilden, wobei dermindestens ein erster Pflegeraum (20; 250) zum Hämodialysebehandlen aus mindestens einer Grundpflegezelle (21; 252) besteht und in mindestens einem Grundmodul (12) montiert ist und mindestens einen Behandlungssitz (80) oder ein Behandlungsbett für einen Patienten enthält, einem Satz technischer Behandlungskomponenten zugeordnet, wobei die technischen Komponenten einen Hämodialysegenerator (81), der zum Herstellen einer Behandlungslösung durch Verdünnen mindestens eines Konzentrats zum Anpassen an den zu behandelnden Patienten eingerichtet ist, eine Vorrichtung zum Anschliessen an ein Stromversorgungsnetz, eine Vorrichtung zum Anschliessen an ein IT-Netzwerk, eine Vorrichtung zum Anschliessen an eine Versorgung für Wasser für die Hämodialyse, eine Vorrichtung zum Anschliessen an eine Versorgung für Konzentrat für die Hämodialyse und eine Abfallsammelvorrichtung umfassen, und wobei in der mindestens einen Grundpflegezelle (21; 252) der Hämodialysegenerator (81) eingerichtet ist, um eine an einen bestimmten Patienten angepasste Lösung realisieren, indem eine vorbestimmte Menge an Konzentraten für die Hämodialyse und ein vorbestimmtes Volumen Wasser für die Hämodialyse entnommen werden, und um das Mischen dieser Komponenten im Hinblick auf die Herstellung und Injektion der für den spezifischen Patienten geeigneten Lösung für die Dauer der Behandlung durchzuführen,
**dadurch gekennzeichnet, dass**
mindestens eine Grundpflegezelle (21; 252) für die Hämodialysebehandlung zwei Plätze aufweist und zwei Behandlungssitze (80) oder -betten für die gleichzeitige Behandlung von zwei Patienten enthält, wobei jeder der Sitze oder Betten einem Satz technischer Behandlungskomponenten zugeordnet ist, und dass jedes Grundmodul (12) ein starres Gerüst (61) umfasst, an dem Abschluss- (68) und/oder Trennwände montiert sind, wobei das starre Gerüst in seinem oberen Teil einen leeren Raum (70) zur Aufnahme technischer Komponenten umfasst.

2. Autonome Behandlungseinheit (10) nach Anspruch 1,
**dadurch gekennzeichnet, dass**
der leere Raum (70) mindestens einen Einlasskanal zum Verteilen gereinigter Luft und mindestens einen Auslasskanal zum Ablassen von Umgebungsluft sowie einen beheizten oder gekühlten Flüssigkeitszirkulationskreislauf enthält, um eine Klimatisierung im Inneren des Behandlungsraums zu gewährleisten.

3. Autonome Behandlungseinheit (10) nach Anspruch 1,
**dadurch gekennzeichnet, dass**
der Pflegeraum (20) im Wesentlichen in der Mitte der Empfangsstruktur (11) angeordnet ist, die einerseits einen ersten Satz von Grundmodulen (12), die erste spezielle Räume bilden, die zusammengestellt sind, um als Patientenempfangsraum, Verwaltungsraum, medizinischer Versorgungsraum ausgestattet zu werden, und einen zweiten Satz von Grundmodulen umfasst, die zweite spezielle Räume bilden, die zusammengestellt sind, um als Lagerraum für Konzentratlösungen, Wasseraufbereitungsraum, als Lagerraum für pharmazeutische Produkte, als Raum zum Management der elektrischen Energie und als Raum zur Abfallbehandlung/-entsorgung ausgestattet zu werden.

4. Autonome Behandlungseinheit (10) nach Anspruch 1,
**dadurch gekennzeichnet, dass**
der Pflegeraum (20) für die Hämodialysebehandlung mindestens ein Grundmodul (12) umfasst, das der Behandlung von Patienten von Patienten mit bestimmten Kontaminationen vorgesehen ist, die medizinische Vorsichtsmaßnahmen zum Schutz anderer Patienten und/oder Pflegepersonal erfordern, wobei das Grundmodul einen gesicherten Zugang hat.

5. Autonome Behandlungseinheit (10) nach Anspruch 1,
**dadurch gekennzeichnet, dass**
das starre Gerüst (61), an dem Abschluss- und/oder Trennwände angebracht sind, ein Metallgerüst ist und dass die Abschluss- (68) und/oder Trennwände mit Platten aus wärmeisolierten Verbundwerkstoffen hergestellt sind, die mit wasserdichten Oberflächenmaterialien bedeckt sind.

6. Autonome Behandlungseinheit (10) nach Anspruch 1,
**dadurch gekennzeichnet, dass**
die starren Gerüste (61) der nebeneinander angeordneten Grundmodule (12) derart zusammengesetzt sind, dass jeder spezielle Raum eine an seine Funktion angepasste Oberfläche aufweist und die Abschluss- und/oder Trennwände an den starren Gerüsten derart montiert sind, um die Oberfläche, die für die dem Raum zugewiesene Funktion geeignet ist, geometrisch abzugrenzen.

7. Autonome Behandlungseinheit (10) nach Anspruch 1,
**dadurch gekennzeichnet, dass**
jedes der Grundmodule (12) eine doppelte Decke (71) umfasst, die den freien Raum (70) oberhalb der Pflegeräume und der speziellen Räume definiert, wobei der freie Raum (70) für Leitungen und/oder technische Kanäle (84, 85) vorgesehen ist.

8. Autonome Behandlungseinheit (200) nach Anspruch 1,
**dadurch gekennzeichnet, dass**
der Technikraum (210) Mittel (211) zum Aufnehmen und Lagern von Rohstoffen, eine Probenahmeeinheit (212), Wiegemittel (213), Geräte zum Vorbereiten der Lösungen zur Patientenbehandlung (214), eine Filtrationseinheit (215) und eine Fülleinheit (216) für Behälter zur Lagerung der Konzentrate umfasst.

9. Autonome Behandlungseinheit (200) nach Anspruch 1,
**dadurch gekennzeichnet, dass**
der Technikraum (210) in den Behandlungsraum (250) integriert ist.

## Claims

1. Self-contained treatment unit (10; 200) for treating at least one patient by haemodialysis, said unit (10; 200) comprising at least one care section equipped with a dialysis generator, said dialysis generator being arranged to prepare a treatment solution adapted to said at least one patient, said treatment solution being prepared by dilution, with haemodialysis water, of at least one concentrate composed of solid compounds that are soluble in the water, said solid compounds comprising at least sodium chloride (NaCI), potassium chloride (KCI), calcium chloride (CaCl2) and magnesium chloride (MgCl2), said self-contained treatment unit (10; 200) is constructed in a reception structure (11) grouping a plurality of elementary modules (12) which are configured substantially identically and juxtaposed and arranged internally, individually or in combination, to form a first care room (20; 250) assigned to the haemodialysis treatment of said at least one patient, dedicated rooms arranged respectively for the reception of the patients, the administrative management of the patients, the medical management of the patients, and a second technical room (30; 210) for the preparation and the storage of the concentrates, the preparation and the control of the fluids used in said first care room (20; 250), and/or the reprocessing and/or disposal of the residual waste and effluents generated by the treatment of the patients, said at least one first care room (20; 250) for haemodialysis treatment is composed of at least one basic care cell (21; 252) and is set up in at least one elementary module (12) and contains at least one treatment seat (80) or bed for a patient, associated with a set of technical treatment components, said technical components comprising a haemodialysis generator (81) arranged to prepare a treatment solution by diluting said at least one concentrate in order to adapt the latter to said patient to be treated, a device for connection to an electricity supply network, a device for connection to a data processing network, a device for connection to a haemodialysis water supply, a device for connection to a haemodialysis concentrate supply, and a waste collection device, and, in said at least one basic care cell (21; 252), said haemodialysis generator (81) is arranged to produce a solution adapted to a specific patient by taking a predetermined quantity of haemodialysis concentrates and a predetermined volume of water for haemodialysis and mixing these components in order to prepare and inject said solution adapted to said specific patient throughout the duration of the treatment, **characterized in that** said at least one basic care cell (21; 252) for haemodialysis treatment has two places and contains two treatment seats (80) or beds for the simultaneous treatment of two patients, each of said seats or beds being associated with a set of technical treatment components, and **in that** each elementary module (12) comprises a rigid frame (61) on which closing and/or separating walls (68) are mounted, in which said rigid frame comprises, in its upper part, an empty space (70) for containing technical components.

2. Self-contained treatment unit (10) according to Claim 1, **characterized in that** said empty space (70) contains at least one purified air supply duct and at least one ambient air exhaust duct, and also a circulation circuit for heated or refrigerated liquid in order to provide air conditioning inside said treatment room.

3. Self-contained treatment unit (10) according to Claim 1, **characterized in that** said care room (20) is arranged substantially at the centre of said reception structure (11), which comprises, on the one hand, a first set of elementary modules (12) constituting first dedicated rooms, assembled to be arranged as a patient reception room, as a formalities room, as a medical administration room, and a second set of elementary modules constituting second dedicated rooms, assembled to be arranged as a concentrate solutions storage room, as a water treatment room, as a pharmaceutical products storage room, as an electrical energy management room, and as a waste processing/disposal room.

4. Self-contained treatment unit (10) according to Claim 1, **characterized in that** said care room (20) for haemodialysis treatment comprises at least one elementary module (12) assigned to the treatment of patients who have particular infections that require medical precautions in order to protect the other patients and/or the healthcare providers, said elementary module having a secured access.

5. Self-contained treatment unit (10) according to Claim 1, **characterized in that** said rigid frame (61) on which closing and/or separating walls are mounted is a metal frame, and **in that** said closing and/or separating walls (68) are formed with thermally insulated composite material panels covered with impervious finishing materials.

6. Self-contained treatment unit (10) according to Claim 1, **characterized in that** the rigid frames (61) of the juxtaposed elementary modules (12) are assembled in such a way that each dedicated room has a surface adapted to its function, and the closing and/or separating walls are mounted on said rigid frames in such a way as to delimit geometrically said surface adapted to the function assigned to said room.

7. Self-contained treatment unit (10) according to Claim 1, **characterized in that** each of the elementary modules (12) comprises a double ceiling (71) that defines said free space (70) at the top of the care rooms and of the dedicated rooms, said free space (70) being used for technical ducts and/or shafts (84, 85).

8. Self-contained treatment unit (200) according to Claim 1, **characterized in that** said technical room (210) comprises means (211) for receiving and storing raw materials, a sampling unit (212), weighing means (213), equipment (214) for preparing the treatment solutions for the patients, a filtration unit (215), and a unit (216) for filling of containers intended to store the concentrates.

9. Self-contained treatment unit (200) according to Claim 1, **characterized in that** said technical room (210) is integrated in the treatment room (250).
